# DEMANDE DE BREVET EUROPEEN

(11) **EP 0 542 632 A1**
(43) Date de publication de la demande: **19.05.1993**
(21) Numéro de dépôt: 92403060.4
(22) Date de dépôt: 13.11.1992
(51) Int. Cl.: A61K 31/07, A61K 33/04, A61K 31/375, A61K 31/355, A61K 31/195

(54) **Utilisation du beta-carotène dans le traitement de fond de l'asthme**

(30) Priorité: 14.11.1991 FR 9114008
(71) Demandeur: LABORATOIRES ARKOPHARMA Société Anonyme, F-06510 Carros (FR)
(72) Inventeur: Rombi, Max, F-06000 Nice (FR)
(74) Mandataire: Bourgognon, Jean-Marie

(57) **Abrégé**

Le traitement comprend l'utilisation du β-carotène pour la préparation d'un médicament des affections respiratoires.

## Description

La présente invention est relative au traitement de fond de l'asthme.

Dans le brevet WO-A- 8900427 on revendique des propriétés non démontrées dans de nombreuses pathologies parmi lesquelles la revendication 26 qui stipule: "Traitement des troubles de la fonction bronchique et pulmonaire, protection de la muqueuse bronchique, des alvéoles pulmonaires, des macrophages alvéolaires et du facteur surfactant". Ces indications, très larges en elles-mêmes, concernent essentiellement les problèmes d'hypersécrétion de mucus au niveau broncho-pulmonaire, dont les complications classiques sont la bronchite chronique, les BPCO et les infections broncho-pulmonaires.

Par ailleurs, dans ce document, la voie d'administration revendiquée est la voie intraveineuse (page 17). Cette voie d'administration peut être envisageable chez les patients cancéreux, mais n'est pas concevable pour le traitement de fond de l'asthme où la durée de traitement est très longue.

Les principales affections respiratoires sont, outre le cancer du poumon, l'asthme et l'emphysème.

L'emphysème est une pathologie bronchique : c'est un état caractérisé par une distension permanente des espaces aériens distaux, situés au-delà des bronchioles terminales avec destruction centrale du lobule. Il se produit une augmentation des résistances responsables de l'obstruction organique des petites voies aériennes. Les lésions de bronchite évoluent à partir du centre du lobule. Il s'ensuit un syndrome ventilatoire obstructif. Le traitement de fond met en oeuvre des vaccins antigrippaux et antipneumococciques par immunorodulateurs, une kinésithérapie respiratoire. Le traitement symptomatique consiste en une antibiothérapie et en l'utilisation d'expectorants et de régulateurs de la sécrétion du mucus.
L'asthme est un syndrome clinique caractérisé par un rétrécissement diffus des voies aériennes bronchiques, réversible spontanément ou sous l'effet d'un traitement bronchodilateur, en relation avec une hyperréactivité bronchique à différents stimuli. Les causes de cette hyperréactivité sont un dysfonctionnement des systèmes sympathique et parasympathique et des anomalies de la muqueuse. Le traitement de fond de l'asthme peut comporter:
- un traitement anti-infectieux (éviction des foyers ORL et stomatologiques)
- un traitement à visée antiallergique (cromoglycate de sodium, nédocromil)
- un traitement de la composante psychique (anxiolytiques)
- un traitement par le Zaditen (qui possède des propriétés antianaphylactiques et une action spécifique antihistaminique).

Le traitement symptomatique fait appel aux bronchodilatateurs: β2-mimétiques, théophylline et corticoïdes.

L'invention a pour objet l'utilisation du β-carotène pour la préparation d'un médicament de traitement de fond de l'asthme par voie orale.

Le médicament peut comprendre de 5 à 50 mg de β-carotène par unité de prise, la posologie quotidienne étant également de 5 à 50 mg.

De préférence, le β-carotène est additionné d'un dérivé de sélénium. Le médicament comprend notamment de 50 à 200 µg de sélénium par unité de prise, la posologie quotidienne pouvant être égale à ces valeurs.

On peut utiliser tous les dérivés organiques et minéraux du sélénium tels que des sélénites, des sélénates, des levures séléniées, de la sélénométhionine, de la sélénocystéine. On préfère le sélénite de sodium en raison de sa meilleure biodisponibilité.

Il est recommandé d'ajouter au médicament suivant l'invention de la vitamine C ou l'un de ses dérivés, à raison de 60 à 10000 mg par unité de prise, la dose quotidienne pouvant être de 60 à 1000 mg par jour. On peut utiliser n'importe quel dérivé de la vitamine C, dont notamment l'ascorbate de sodium qui est très stable.

Il est recommandé d'ajouter au médicament suivant l'invention de la vitamine E ou l'un de ses dérivés à raison de 10 à 1000 mg par unité de prise, la posologie quotidienne étant également de 10 à 1000 mg.

On peut utiliser l'ensemble des dérivés de la vitamine E avec une préférence particulière pour l'acétate de DL-α-tocophérol qui présente l'activité pharmacologique la plus élevée.

Enfin, il est recommandé d'additionner les principes actifs du médicament suivant l'invention de glutathion ou d'un précurseur de glutathion tels que cystine et ses dérivés, cystéine et ses dérivés, méthionine et ses dérivés, à raison de 25 à 100 mg par unité de prise pour une posologie quotidienne comprise également entre 25 et 100 mg.

Le médicament suivant l'invention est formulé, notamment pour l'administration par voie orale, sous forme de gélules comprenant de préférence tous les constituants actifs précités, ainsi qu'un véhicule ou excipient pharmaceutique, le rapport pondéral des principes actifs à l'excipient étant compris entre 3 et 6 et, de préférence, entre 4, 6 et 5.

Chacun des principes actifs constituant l'association suivant l'invention est un constituant connu qui n'est pas toxique aux doses auxquelles il est administré.

L'étude clinique suivante illustre l'efficacité du médicament suivant l'invention.

On a constitué deux groupes de 18 sujets chacun, de 18 à 70 ans, présentant un asthme de type III (plus d'une crise d'asthme par semaine) ou IV (asthme à dyspnée continue) selon la classification de Charpin. On administre deux gélules par jour de placebo ou de la composition selon l'invention répartie en deux prises pendant deux mois. La formulation de la composition selon l'invention est la suivante.

| | |
|---|---|
| Vitamine C | 200 mg |
| Acétate de tocophérol | 200 mg |
| β-carotène | 6 mg |
| Sélénium (sous forme de sélénite de sodium) | 50 mg |
| DL-méthionine | 25 mg |

On étudie les paramètres fonctionnels respiratoires suivants.
- le volume expiré maximum par seconde (VEMS) à J0, J30 et J60 (c'est-à-dire au début de l'étude, 30 jours après le début de l'étude et 60 jours après le début de l'étude ;
- la capacité vitale (CV) à J0, J30 et J60 ;
- le débit expiratoire de pointe (DEP) à J0, J30 et J60 ;
- la résistance spécifique des voies aériennes (SRVA) à J0, J30 et J60 ;
- la dose liminaire de carbachol nécessaire pour augmenter de 100 % la résistance spécifique (DL) à J0 et J60.

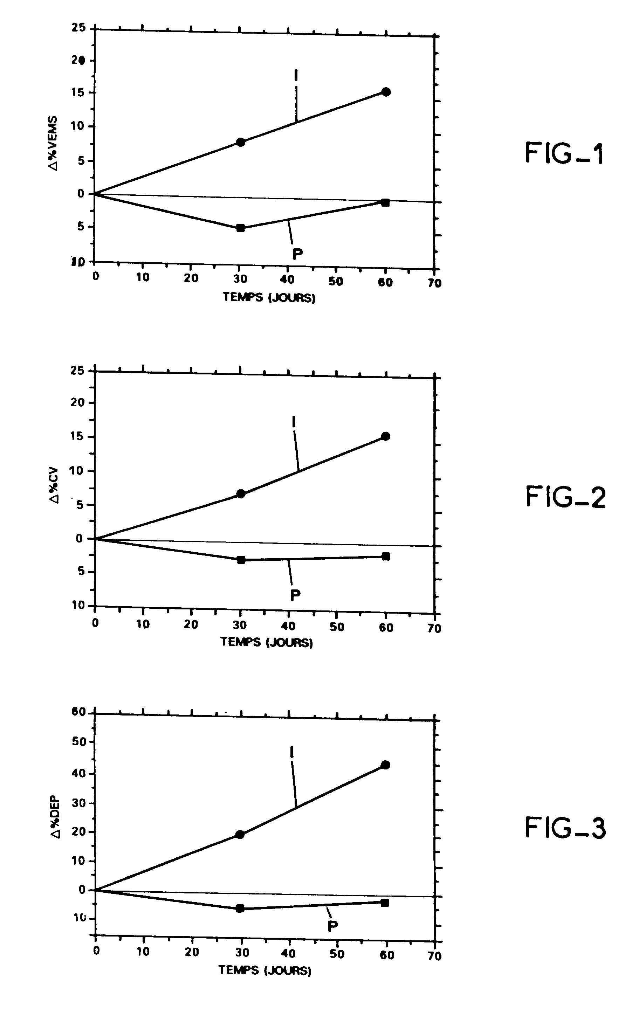

Les résultats obtenus sont illustrés aux figures 1 à 3 qui donnent la variation du VEMS, de la CV et du DEP, en fonction du temps, les courbes I correspondant au groupe ayant reçu le médicament suivant l'invention et les courbes P au groupe ayant reçu le placebo.

Dans le groupe placebo, le VEMS reste constant pendant les deux mois de traitement, alors que dans le groupe ayant reçu le médicament suivant l'invention il augmente régulièrement. Après deux mois de traitement, le VEMS augmente significativement de 26 % (p=0,004) dans le groupe ayant reçu le médicament suivant l'invention.

Dans le groupe placebo, la capacité vitale reste constante pendant les deux mois de l'étude, alors que dans le groupe ayant reçu le médicament suivant l'invention elle augmente régulièrement, pour atteindre 16 % au soixantième jour de traitement. Cette augmentation est statistiquement significative (p = 0,009).

La variation du DEP au cours des deux mois de traitement est similaire à celle du VEMS : stable dans le groupe placebo, elle augmente dans le groupe ayant reçu le médicament suivant l'invention, pour atteindre 46 % après deux mois de traitement (p = 0,001).

La SRVA baisse de 19 % dans le groupe ayant reçu le médicament suivant l'invention.

La dose liminaire de carbachol, qui provoque une élévation de 100 % de la SRVA, augmente beaucoup après deux mois de traitement par le médicament suivant l'invention (+58 %), alors qu'elle reste stable dans le groupe placebo.

## Revendications

1. Utilisation du β-carotène pour la préparation d'un médicament de traitement de fond par voie orale de l'asthme.

2. Utilisation suivant la revendication 1, caractérisée en ce que le médicament comprend de 5 à 50 mg de β-carotène.

3. Utilisation suivant la revendication 1 ou 2, caractérisée en ce que le β-carotène est additionné d'un dérivé de sélénium.

4. Utilisation suivant la revendication 3, caractérisée en ce que le médicament comprend 50 à 200 µg de sélénium.

5. Utilisation suivant l'une des revendications précédentes, caractérisée en ce que le médicament comprend de la vitamine C.

6. Utilisation suivant la revendication 5, caractérisée en ce que le médicament comprend de 60 à 1000 mg de vitamine C.

7. Utilisation suivant l'une des revendications précédentes, caractérisée en ce que le médicament comprend de la vitamine E ou l'un de ses dérivés.

8. Utilisation suivant la revendication 7, caractérisée en ce que le médicament comprend 10 à 1000 mg de vitamine E ou de l'un de ses dérivés.

9. Utilisation suivant l'une des revendications précédentes, caractérisée en ce que le médicament comprend de la méthionine.

10. Utilisation suivant la revendication 9, caractérisée en ce que le médicament comprend de 25 à 100 mg de méthionine.
